# EUROPÄISCHE PATENTSCHRIFT

(11) **EP 3 571 497 B1**
(45) Veröffentlichungstag und Bekanntmachung des Hinweises auf die Patenterteilung: **17.11.2021**
(21) Anmeldenummer: 18701698.5
(22) Anmeldetag: 18.01.2018
(51) Int. Cl.: G01N 27/333, G01N 33/02, G01N 33/18, G01N 33/24

(54) **SYSTEM ZUR BESTIMMUNG ODER ÜBERWACHUNG EINER ZUSTANDSGRÖSSE EINES MESSOBJEKTS UND ENTSPRECHENDES VERFAHREN**
SYSTEM FOR DETERMINING OR MONITORING A STATE VARIABLE OF A MEASUREMENT OBJECT AND CORRESPONDING METHOD
SYSTÈME POUR DÉTERMINER OU SURVEILLER UNE GRANDEUR D'ÉTAT D'UN OBJET À MESURER ET PROCÉDÉ CORRESPONDANT

(30) Priorität: 19.01.2017 DE 102017200884; 03.07.2017 DE 102017211282
(43) Veröffentlichungstag der Anmeldung: 27.11.2019
(73) Patentinhaber: Fraunhofer-Gesellschaft zur Förderung der angewandten Forschung e.V., 80686 München (DE)
(72) Erfinder: JANK, Michael, 91058 Erlangen (DE); OERTEL, Susanne, 96175 Pettstadt (DE); ZÖRNER, Alicia Marion, 91058 Erlangen (DE); THIEME, Wolfgang, 90571 Schwaig (DE); HECKEL, Thomas, 91058 Erlangen (DE); JOFFE, Christopher, 91056 Erlangen (DE); RENNER, Esther Ann, 91058 Erlangen (DE); HOFMANN, Christian, 90425 Nürnberg (DE); LANG, Nadine Ramona, 91054 Erlangen (DE); STRUCK, Matthias, 91058 Erlangen (DE); ENDRUSCHAT, Achim, 92367 Pilsach (DE); GERSTNER, Holger, 91058 Erlangen (DE)
(74) Vertreter: Zinkler, Franz
(86) Internationale Anmeldenummer: PCT/EP2018/051191
(87) Internationale Veröffentlichungsnummer: WO 2018/134297

(56) Entgegenhaltungen:
- EP-A1- 2 980 576
- WO-A1-2014/096844
- WO-A1-2014/113460
- WO-A1-2016/083649
- WO-A1-2016/090176
- US-A1- 2015 087 072
- US-A1- 2016 169 855
- US-A1- 2016 327 511
- US-B1- 9 046 461

## Beschreibung

Die Erfindung bezieht sich auf ein System zur Bestimmung und/oder Überwachung mindestens einer Zustandsgröße eines Messobjekts. Weiterhin bezieht sich die Erfindung auf ein Verfahren zur Bestimmung und/oder Überwachung mindestens einer Zustandsgröße eines Messobjekts.

Aufgrund von gesetzlicher Vorlagen ist es z. B. in der Agrarwirtschaft erforderlich, den Zustand von Böden oder auch des Gewässers zu überwachen bzw. zu kontrollieren. Dies dient der Erhaltung der Wasserqualität oder um Überdüngung zu verhindern.

Für die Untersuchung von Böden und Gewässer sind zwei Methoden bekannt: In einer Variante wird eine Probe des Bodens bzw. des Wassers genommen und an ein Labor geschickt. Dies ist zwar genau, jedoch auch teuer. Zudem dauert es eine gewisse Zeit, bis die Ergebnisse vorliegen. Überdies besteht die Möglichkeit, dass die Proben durch den Versand unbrauchbar, kontaminiert oder im Labor vertauscht werden. Alternativ kann direkt am Messobjekt mit einem Teststäbchen ein Abstrich genommen werden. Dies ergibt jedoch nur eine grobe Einschätzung.

Die US 2016 / 169 855 A1 beschreibt ein Analysesystem, das dem sogenannten Precision Farming zugeordnet werden kann. Ein im Boden vergrabenes Datenakquise- und Analysesystem kommuniziert mit einer Vielzahl von im Boden vergrabenen Sensoren. Die Daten der Bodensensoren werden mit Daten eines über den Boden hinwegfliegenden unbemannten Flugobjekts kombiniert, um hierüber Aussagen über den Bodenzustand zu treffen, und zu eruieren, ob korrektive Maßnahmen erforderlich sind.

Die US 9 046 461 B1 beschreibt drahtlose Sensoren zur Überwachung von Umgebungsparametern. Die Sensoren, und insbesondere die Empfänger, befinden sich oberhalb des Bodens und sind in einer Mesh-Topologie angeordnet. Da sich Umgebungsbedingungen im Boden, wie beispielsweise der Feuchtigkeitsgehalt, ändern können, führt dies auch zu einer Änderung des Hochfrequenzverhaltens der Sensoren. Die US 9 046 461 B1 beschreibt ein Verfahren, um diese Änderungen zu kompensieren.

Die Aufgabe der Erfindung besteht darin, eine möglichst einfache und zuverlässige Möglichkeit zur Kontrolle einer Zustandsgröße eines Messobjekts zu ermöglichen.

Die Erfindung löst die Aufgabe durch das im Anspruch 1 offenbarte System zur Bestimmung und/oder Überwachung mindestens einer Zustandsgröße eines Messobjekts.

Das System weist mindestens einen Analysesensor, eine Steuervorrichtung und eine Auslesevorrichtung auf. Der Analysesensor ist ausgestaltet, um zum Verbleib in das Messobjekt einbringbar zu sein. Der Analysesensor ist ausgestaltet, um auf mindestens eine lonenart sensitiv zu sein und ausgehend von einer Konzentration der lonenart im Messobjekt Messdaten zu erzeugen. Die Steuervorrichtung ist ausgestaltet, um den Analysesensor in Bezug auf das Erzeugen der Messdaten zu steuern. Die Auslesevorrichtung ist ausgestaltet, um die Messdaten aus dem Analysesensor auszulesen.

Der Analysesensor erlaubt somit insbesondere die Messung von Ionen, wobei der Analysesensor spezifisch vorzugsweise auf wenigstens - alternativ nur - eine lonenart reagiert. Die lonenart steht dabei in mittelbarem oder unmittelbaren Zusammenhang mit der zu bestimmenden und/oder zu überwachenden Zustandsgröße des Messobjekts, sodass auch die Messdaten des Analysesensors unmittelbar oder mittelbar im Zusammenhang mit der Zustandsgröße stehen.

Der Analysesensor ist derartig ausgestaltet, dass er in das Messobjekt eingebracht wird, um dort zu verbleiben. Es handelt sich somit nicht um eine einmalige Messung, sondern es wird ein dauerhaftes Messsystem geschaffen.

Je nach Ausgestaltung schafft das System eine Möglichkeit einer kontinuierlichen, schnellen, echtzeitfähigen Messung von Ionen in einer Messsubstanz des Messobjekts. Dies dient z. B. zur Kontrolle von Böden oder der Wasserqualität. Je nach Ausgestaltung - insbesondere unter Verwendung einer Auswertevorrichtung - ergibt sich eine direkte, schnelle Auswertung oder sogar eine Rückmeldung in Echtzeit. Zudem ergeben sich die Vorteile einer direkten Probensammlung und einer direkten Auswertung. Werden mehrere Analysesensoren verwendet, so lassen sich beispielweise auch große Agrarflächen ohne ständige Begleitung durch Personal überwachen.

Es folgen drei Varianten für das Messobjekt, dessen Zustandsgröße es zu ermitteln oder zu überwachen gilt.

In einer Ausgestaltung handelt es sich bei dem Messobjekt um einen Boden. Der Boden ist beispielsweise ein Acker, eine Wiese oder ein Feld.

In einer weiteren Ausgestaltung handelt es sich bei dem Messobjekt um ein Gewässer. Das Gewässer ist beispielsweise ein Fluss, ein Teich, eine See oder ein Klärbecken usw. Der Analysesensor oder ggf. die Analysesensoren befinden sich dabei beispielsweise in Wasserleitungen, Wasserhähnen, Hauswasserwerken, Wasserwerken, Brunnen, Tiefbrunnen, Pumpen, Heizanlagen, Wasseraufbereitern, Wasserfiltern, Wasseruhren, Schwimmbecken, Stauseen, Seen- und Gewässer, Flüsse, Bäche usw. Anwendungen beziehen sich jedoch auch auf den Privatgebrauch, z. B. Schwimmbecken. In einer anderen Ausgestaltung handelt es sich bei dem Messobjekt um ein landwirtschaftliches Produkt. Das landwirtschaftliche Produkt ist z. B. Obst oder Gemüse oder es handelt sich um eine Biomasse zur Gewinnung von Energie. Weiterhin kann es sich um Futtermittel in Form von Silage handeln.

Eine Zustandsgröße ist beispielsweise der Nitratgehalt, für den es in der Landwirtschaft gesetzliche Vorgaben gibt. So ist z. B. die Nitratkonzentration in Brunnen- oder Trinkwasseranlagen regelmäßig zu kontrollieren, wobei vorgegebene Grenzwerte nicht überschritten werden dürfen. Die Düngung von Äckern darf beispielsweise im Rahmen der Stickstoffbilanz nur bis zu einer vorgegebenen Maximalgrenze pro Fläche und pro Jahr erfolgen.

Weitere Zustandsgrößen sind z. B. die Ammoniumkonzentration bzw. die Ammoniakkonzentration: Diese Parameter eignen sich sehr gut als Indikatoren für Gewässerverunreinigungen durch häusliche Abwässer und landwirtschaftliche Produktion. Bei Ammonium handelt es sich um einen ungiftigen Pflanzennährstoff. Ammoniak hingegen ist ein farbloses, stechend riechendes giftiges Gas. Beide Formen kommen im Wasser nebeneinander vor. Wichtig dabei ist an dieser Stelle der Bezug zum pH-Wert. Der pH-Wert wird dabei beispielsweise über einen im Folgenden vorgestellten Zusatz-Sensor ermittelt. Um den Neutralpunkt 7,0 liegt ein ausgeglichenes Verhältnis zwischen Ammonium und Ammoniak vor. Verschiebt sich der pH-Wert ins alkalische (basische) Milieu, so wird aus den Ammonium-Ionen das Ammoniak freigesetzt. In stark basischen Lösungen liegt freies Ammoniak vor, welches in höheren Konzentrationen für jegliches Leben im Gewässer tödlich ist. Weitere Zustandsgrößen sind z. B. die Konzentration und/oder die Art der Säuretypen. Beim Beispiel der Silage liefert die Überwachung des pH-Werts in der Regel alleine keine ausreichende Aussage über die Gärqualität. Die Erfassung von mehreren Säuretypen (z. B. Butter-, Essig- und Milchsäure) ist deshalb erforderlich.

Mögliche Anwendungen des Systems in der Landwirtschaft sind beispielsweise:
- Die Kontrolle des Stickstoffgehaltes in Form von Nitratgrenzwerte oder Bestimmen des optimalen Aussaatzeitpunkts usw., wobei mindestens ein Analysesensor in einen feuchten Boden eingebracht wird.
- Überwachung von Wasserspeichern, die z. B. landwirtschaftlich genutzt werden.
- Überwachung des Grundwasservorkommens, das durch Agrarflächen beeinflusst wird.
- Überwachung von Gülletanks - als Beispiel für Träger von landwirtschaftlichen Produkten - zur Abklärung von Düngergehalt vor dem Aufbringen auf das Feld.
- Kontrolle von Trink- und Brunnenwasser (gesetzliche Vorschrift auch für landwirtschaftliche Betriebe).
- Optimiertes Düngemanagement.
- Überwachung von Silage-Behälter.

Erfindungsgemäß sind mehrere Analysesensoren vorhanden. Die somit mindestens zwei Analysesensoren befinden sich dabei am gleichen oder an unterschiedlichen Orten innerhalb des Messobjekts. Außerdem werden die Analysesensoren über mindestens einen gemeinsamen Knoten ausgelesen. In einer Ausgestaltung kann die Auslesevorrichtung dieser gemeinsame Knoten sein oder zumindest einen solchen gemeinsamen Knoten, beispielsweise in Form einer Ausleseeinheit als Teil der Auslesevorrichtung, aufweisen. Der gemeinsame Knoten ist hierbei ausgestaltet, um zum Verbleib in das Messobjekt einbringbar zu sein. Somit wären die Analysesensoren und der gemeinsame Knoten zum Auslesen der Analysesensoren in dem Messobjekt eingebracht, wobei die Analysesensoren, die im Messobjekt verteilt sein können, mit dem zentral im Messobjekt angeordneten gemeinsamen Knoten innerhalb des Messobjekts kommunizieren können.

In einer Ausgestaltung ist vorgesehen, dass die Analysesensoren auf die gleiche lonenart sensitiv sind. Alternativ oder ergänzend ist in einer Ausgestaltung vorgesehen, dass die Analysesensoren auf unterschiedliche lonenarten sensitiv sind. In einer Ausgestaltung sind mindestens zwei Analysesensoren auf die gleiche lonenart und ist wenigstens ein Analysesensor auf eine andere lonenart sensitiv.

In einer weiteren Ausgestaltung ist wenigstens ein Analysesensor auf mindestens zwei lonenarten sensitiv. Ein solcher Multisensor liefert Messdaten über mehrere lonenarten, um z. B. die Wechselwirkung verschiedener Ionen zu detektieren. Dabei lassen sich die Messdaten über entsprechende Algorithmen interpretieren.

In einer Ausgestaltung weist das System mindestens eine Speichervorrichtung auf.

Die Speichervorrichtung dient in einer Ausgestaltung der Hinterlegung von Auswertedaten und/oder von Referenzdaten für die Auswertung der Messdaten des mindestens einen Analysesensors. Die Speichervorrichtung kann ausgestaltet sein, um zum Verbleib in das Messobjekt einbringbar zu sein. Die Speichervorrichtung kann mit der Auslesevorrichtung verbunden und mit dieser zusammen in dem Messobjekt eingebracht sein.

In einer alternativen oder ergänzenden Ausgestaltung ist die Speichervorrichtung ausgestaltet, um die Messdaten und/oder von den Messdaten abgeleitete Daten zu speichern. In einer Ausgestaltung ist die Auslesevorrichtung ausgestaltet, um Messdaten aus dem mindestens einen Analysesensor auszulesen und in der Speichervorrichtung abzuspeichern. Die von den Messdaten abgeleiteten Daten sind z. B. anhand von Referenzdaten aus den primären Messdaten (z. B. ein Spannungswert) abgeleitete Aussagen über die Ionen bzw. über das Messmedium, in dem sich der Analysesensor befindet (z. B. die Konzentration der Ionen). Alternativ oder ergänzend sind die abgeleiteten Daten ein Maß für die Änderungen der Messdaten des mindestens einen Analysesensors oder ein Maß für die Abweichung zwischen den Messdaten mehrerer Analysesensoren.

Gemäß einer Ausgestaltung weist das System mindestens eine Auswertevorrichtung auf. In einer Ausgestaltung ist die Auswertevorrichtung ausgestaltet, um die Messdaten des mindestens einen Analysesensors oder alternativ der Analysesensoren im Hinblick auf die Zustandsgröße auszuwerten und/oder von den Messdaten Daten abzuleiten. In einer Ausgestaltung ist die Auswertevorrichtung mit der Speichervorrichtung und/oder mit der Steuervorrichtung und/oder mit der Auslesevorrichtung verbunden. Die Auswertevorrichtung kann ausgestaltet sein, um zum Verbleib in das Messobjekt einbringbar zu sein. In einer Ausgestaltung kann die Auswertevorrichtung zusammen mit der Speichervorrichtung und/oder der Auslesevorrichtung in dem Messobjekt eingebracht sein. Die Auswertung beinhaltet in einer Ausgestaltung die Ermittlung einer flächenbezogenen Konzentration und/oder einer Konzentrationsänderung. Werden mehrere Analysesensoren verwendet, so beinhaltet in einer Ausgestaltung die Auswertung die Ermittlung einer Konzentrationsverteilung.

Eine Ausgestaltung sieht vor, dass das System mindestens einen Zusatz-Sensor aufweist. In einer Ausgestaltung ist der Zusatz-Sensor ausgestaltet, um Messdaten mindestens in Abhängigkeit von einer Temperatur, einem Füllstand, einem Durchfluss (Massen- und/oder Volumendurchfluss), einem pH-Wert, einem elektrischen Widerstand, einer elektrischen Leitfähigkeit, einem Anteil eines Gases, einem Anteil von Sauerstoff (als einem ausgesuchten Gas) oder einer Fließgeschwindigkeit zu erzeugen. Der Zusatzsensor unterscheidet sich somit von den Analysesensoren und ist vorzugsweise nicht abhängig von einer speziellen lonenart. In einer Ausgestaltung beziehen sich die Messdaten des Zusatzsensors auf das Messobjekt (z. B. die Temperatur oder der Füllstand in einem Behälter) selbst und in einer alternativen Ausgestaltung auf die Umgebung des Messobjekts (z. B. die Menge des sich in das Messobjekt ergießenden Wassers).

Gemäß der Erfindung weist das System mindestens eine Energiequelle auf. Die Energiequelle ist dabei ausgestaltet, um mindestens einen Analysesensor mit Energie zu versorgen. Alternativ oder ergänzend dient die Energiequelle der Energieversorgung mindestens der Auslesevorrichtung, der Auswertevorrichtung, der Steuervorrichtung oder der Speichervorrichtung.

Die folgenden Ausgestaltungen beziehen sich auf die konkrete Ausgestaltung der Energiequelle.

Eine Ausgestaltung sieht vor, dass die Energiequelle mindestens eine Batterie aufweist.

Erfindungsgemäß weist die Energiequelle jedoch stets mindestens einen Akku auf. In der erfindungsgemäßen Ausgestaltung ist die Energiequelle derartig ausgestaltet, dass der Akku berührungslos aufladbar ist. Erfindungsgemäß erfolgt die Aufladung durch induktives Wirkprinzip, wobei an den Akku eine entsprechende elektrische Schaltung und/oder Komponenten zur Einkopplung und Wandlung der Energie für das Aufladen des Akkus angebracht sind.

Gemäß einer ergänzenden Ausgestaltung ist die Energiequelle ausgestaltet, um mittels "Energy Harvesting" Energie zu erzeugen. Hierfür wird beispielweise Windenergie oder eine Temperaturdifferenz ausgenutzt.

In einer Ausgestaltung ist vorgesehen, dass mindestens ein Analysesensor und die Auslesevorrichtung kabellos und/oder durch elektromagnetische Wellen miteinander in Bezug auf Datenübertragung verbunden sind. In dieser Ausgestaltung besteht somit insbesondere keine Kabelverbindung zwischen mindestens einem Analysesensor und der Auslesevorrichtung. Alternativ oder ergänzend sind auch mindestens ein Analysesensor und die Steuervorrichtung kabellos miteinander kontaktiert.

Die Daten werden in einer Ausgestaltung insbesondere über kurze Distanzen hinweg per Bluetooth Low Energy übertragen. In einer weiteren Ausgestaltung verfügen die miteinander kommunizierenden Komponenten über Antennen, die auch über weitere Distanzen eine Datenübertragung erlauben.

In einer Ausgestaltung ist die Auslesevorrichtung als mobile Einheit ausgestaltet. In dieser Ausgestaltung wird beispielsweise die Auslesevorrichtung in die Nähe der in dem Messobjekt befindlichen Analysesensoren - und ggf. des mindestens einen Zusatzsensors gebracht - und die Messdaten werden ausgelesen. Die mobile Einheit verfügt vorzugweise über mindestens eine Antenne zum Empfangen der Daten.

Erfindungsgemäß weist die Auslesevorrichtung eine erste, zentrale, Ausleseeinheit auf, die ausgestaltet ist, um zum Verbleib in das Messobjekt eingebracht zu werden. In einer solchen Ausgestaltung liest die in das Messobjekt eingebrachte zentrale erste Ausleseeinheit die ebenfalls in dem Messobjekt befindlichen Analysesensoren zentral aus. In dieser Ausgestaltung weist die Auslesevorrichtung ferner eine zweite, gegebenenfalls mobile, Ausleseeinheit auf. Die mobile zweite Ausleseeinheit ist außerhalb des Messobjekts befindlich und kommuniziert mit der in dem Messobjekt befindlichen zentralen ersten Ausleseeinheit.

Die folgenden Ausgestaltungen beziehen sich auf die konkrete Ausgestaltung des mindestens einen Analysesensors.

Gemäß einer Ausgestaltung weist mindestens ein Analysesensor mindestens eine auf einer - vorzugsweise flexiblen - Folie - z. B. mit Siebdruckpasten - gedruckte ionenselektive Elektrode, eine - vorzugsweise mindestens einen lonophor aufweisende - ionenselektive Membran, eine Referenzelektrode und eine Gegenelektrode auf. Der lonophor dient dabei dem Transport der zugeordneten lonenart durch die - somit ionenselektive - Membran. Dabei ist der Analysesensor vorzugsweise preiswert.

Eine ionenselektive Elektrode (andere Bezeichnungen sind: ionenspezifische oder ionensensitive Elektrode) erlaubt die Messung der Konzentration bzw. der Aktivität eines bestimmten gelösten Ions. Gemessen wird dabei eine elektrische Spannung zwischen der ionenselektiven Elektrode und einer zweiten Elektrode (die Bezugs- bzw. Referenzelektrode). In einer Ausgestaltung besteht die Folie zumindest teilweise aus Polyester (PET oder PEN) oder aus Polyimiden (PI) oder anderen synthetischen Materialien wie Polyurethan (PU) oder aus synthetischen Textilien. Passieren eine Membran unterschiedliche lonenarten, so erlauben entsprechende Kalibrierungsdaten eine Kompensation der Messwerte.

Vorzugweise handelt es sich um einen gedruckten Analysesensor, der gedruckte Arbeits- und Referenzelektroden sowie mindestens eine Passivierungsschicht aufweist. Die Herstellung erfolgt vorzugsweise mit kommerziell erhältlichen Siebdruckpasten, wobei in einer Ausgestaltung eine Optimierung der gedruckten Elektroden mittels Ausheizen bei pastenspezifischen Temperaturen erfolgt. In einer Ausgestaltung sieht die Fertigstellung der Referenzelektrode das Aufbringen einer Mischung aus Polyvinylbutyral, Methanol und Natriumchlorid vor (vgl. T. Guinovart et al., "Potentiometric sensors using cotton yarns, carbon nanotubes and polymeric membranes", Analyst, 2013, 5208 - 5215). In einer Ausgestaltung umfasst die Herstellung des ersten Sensors das Aufbringen des ionenselektiven Materials in Form eines lonophors (beispielsweise Nonactin,Valinomycin, Natriumionophor) auf eine Arbeitselektrode. Dabei ist in einer Ausgestaltung eine Mischung mit einer Matrix (netzwerkbildende Materialien, insbesondere Polymere und vorzugsweise Polyvinylbutyral, PVB, und Polyvinylchlorid, PVC) vorgesehen. In einer Ausgestaltung wird die Menge des lonophors minimiert, um eine kostengünstige Realisierung des Analysesensors zu ermöglichen.

Gemäß einer Ausgestaltung ist mindestens ein Analysesensor ausgestaltet, um auf mindestens eine lonenart in einer feuchten oder nassen Umgebung sensitiv zu sein. Der Analysesensor ist somit beispielsweise ausgestaltet, um in Flüssigkeiten oder feuchten Böden die jeweils relevante Ionen bzw. Stoffe messen zu können.

In einer Ausgestaltung erlaubt mindestens ein Analysesensor eine Messung von mindestens einer der folgenden Substanzen und/oder deren Abbauprodukte: Nitrat, Nitrit, Chlorid, Fluorid, Sulfat, Ammonium, Sauerstoff, Phosphate, Kalium, Natrium oder Kalzium.

Alternativ oder ergänzend erlaubt der Analysesensor eine Messung von mindestens einer Säure und/oder von mindestens einem Abbauprodukt bei einem Gärprozess.

In einer Ausgestaltung ist mindestens ein Analysesensor ausgestaltet, um eine Messung mindestens von einem Biozid, einem Weichmacher, Algen, Pilzen, Sporen, Bakterien, einem Giftstoff, einem Toxin oder eines metallischen Stoffs zu erlauben. Die Messung bezieht sich somit auf eine der vorgenannten Zustandsgrößen.

Dabei ist mindestens ein Analysesensor ausgestaltet, um kontinuierlich Messdaten zu erzeugen oder nur zu vorgebbaren Messzeitpunkten Messdaten zu erzeugen.

In einer Ausgestaltung ist mindestens ein Analysesensor derartig ausgestaltet, dass der Analysesensor biologisch abbaubar ist. Der Analysesensor kann somit im Messobjekt vollständig bis zu seiner Auflösung verbleiben.

In einer Ausgestaltung weist mindestens ein Analysesensor eine Identifizierungseinheit auf. Die Identifizierungseinheit ist in einer Ausgestaltung ein RFID-Tag und ist in einer alternativen Ausgestaltung ein Schild oder ein Aufkleber mit einer Seriennummer.

Weiterhin wird hierin beispielhaft ein Verfahren zur Bestimmung und/oder Überwachung mindestens einer Zustandsgröße eines Messobjekts beschrieben.

Das beispielhaft beschriebene Verfahren umfasst zumindest die folgenden Schritte:
- dass mindestens ein Analysesensor zum Verbleib in das Messobjekt eingebracht wird,
- dass von dem Analysesensor ausgehend von einer Konzentration einer lonenart im Messobjekt Messdaten erzeugt werden,
- dass der Analysesensor in Bezug auf das Erzeugen der Messdaten gesteuert wird, und
- dass die Messdaten aus dem Analysesensor ausgelesen werden.

Die Messdaten stehen dabei insbesondere im Zusammenhang mit der Zustandsgröße und erlauben vorzugsweise eine Auswertung in Bezug auf die Zustandsgröße.

Die oben beschriebenen Ausgestaltungen der Vorrichtung lassen sich dabei auch durch das Verfahren realisieren, so dass die Ausgestaltungen und Ausführungen entsprechend auch für das Verfahren gelten. Daher wird auf Wiederholungen verzichtet.

Im Einzelnen gibt es eine Vielzahl von Möglichkeiten, das erfindungsgemäße System auszugestalten und weiterzubilden.

Es zeigen:
- Fig. 1: eine schematische Darstellung eines Beispiels der Anwendung des Systems als Blockschaltbild,
- Fig. 2: eine schematische Darstellung eines weiteren Anwendungsbeispiels und
- Fig. 3: eine Draufsicht auf einen Analysesensor.

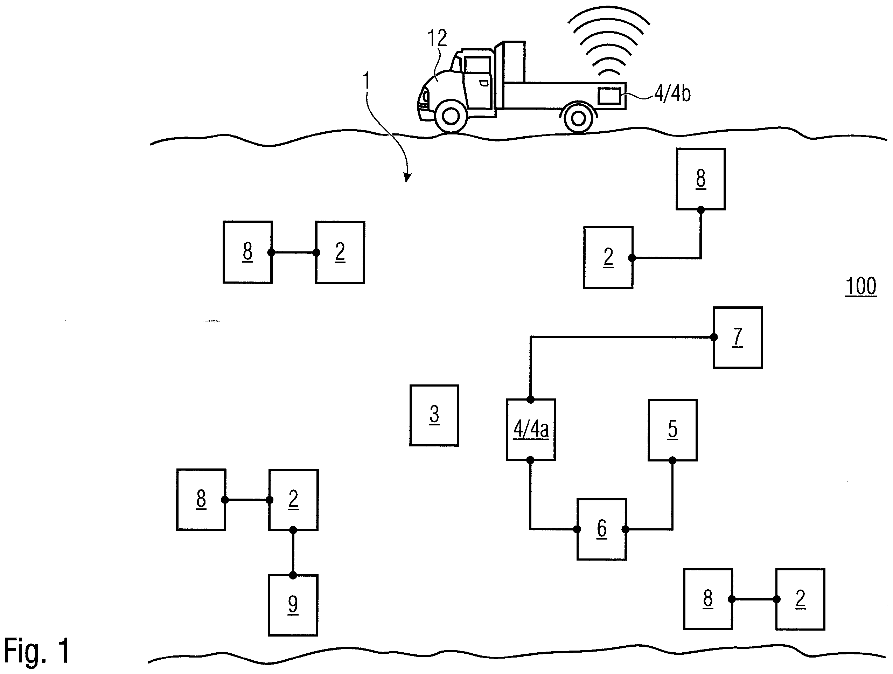

In der Fig. 1 ist schematisch ein Anwendungsbeispiel des Systems 1 dargestellt. Dabei wird eine Zustandsgröße eines Bodens als Messobjekt 100 überwacht bzw. ermittelt. Die Zustandsgröße ist hier als Beispiel die Belastung des Bodens mit einem Dünger.

Das System 1 verfügt dabei in dem Beispiel über vier Analysesensoren 2, die in dem Boden 100 eingebracht sind, um dort dauerhaft zu verbleiben und Messdaten zu erzeugen. Die Analysesensoren 2 reagieren dabei auf Ionen, die sich in einer insbesondere nassen bzw. zumindest feuchten Umgebung befinden.

Für die Messungen und die Kommunikation der Messdaten verfügt in der gezeigten Ausgestaltung jeder Analysesensor 2 über eine Energiequelle 8. Bei den Energiequellen 8 handelt es sich erfindungsgemäß um Akkumulatoren (oder kurz Akkus), die mittels induktivem Wirkprinzip und der zugehörigen elektrischen Schaltung und Komponenten berührungslos aufgeladen werden können. Weiterhin verfügt ein Analysesensor 2 noch über eine Identifizierungseinheit 9, über die beispielsweise zusätzlich mittels GPS-Technologie die Position des Analysesensors 2 zu identifizieren ist. Die anderen Analysesensoren 2 lassen sich beispielsweise über ihre Seriennummern identifizieren.

Die Messungen der Analysesensoren 2 werden durch eine Steuervorrichtung 3 gesteuert, die insbesondere über Funk mit den Analysesensoren 2 kommuniziert und die Messzeitpunkte vorgibt oder auch die Energieversorgung überwacht. Die Steuervorrichtung 3 kann ebenfalls in dem Boden 100 eingebracht sein.

Die Steuervorrichtung 3 ist ausgestaltet, um mit den Analysesensoren 2 zu kommunizieren und eine mittels der Analysesensoren 2 durchzuführende Messung zu initiieren und/oder zu koordinieren.

In einer alternativen Ausgestaltung kann auf die Steuervorrichtung 3 verzichtet werden. In einem solchen Fall könnten die Analysesensoren 2 eine integrierte Steuerung aufweisen. Die integrierte Steuerung könnte dabei ausgestaltet sein, um eine Messung in periodischen zeitlichen Abständen zu initiieren. Dabei könnte es sich beispielsweise um eine in Hardware oder Software implementierte Zeitschaltuhr handeln.

In einem weiteren denkbaren Ausführungsbeispiel könnten die Analysesensoren 2 beispielsweise eine integrierte, intelligente Steuereinheit aufweisen. Eine solche Steuereinheit könnte intelligent (zum Beispiel basierend auf Feedback-Regelkreise, oder einer Statistischen Auswertung früherer Messwerte) entscheiden, wie oft Messwerte erfasst werden sollen. Wenn sich in einem Bodenabschnitt 100 die Messwerte über z.B. ein Jahr hinweg kaum ändern, können zum Beispiel im folgenden Jahr weniger Messungen durchgeführt/protokolliert und Messpunkte aufgezeichnet werden. Falls sich Messwerte hingegen häufiger ändern oder einen großen Wertebereich abdecken, können die Messungen in Zukunft häufiger durchgeführt werden. Die intelligente Steuerung kann auch durch Einbeziehung weiterer Sensordaten, wie zum Beispiel von Niederschlagsmengen-, Temperatur- und Feuchtigkeitssensoren erfolgen, die ebenfalls im Bodenabschnitt 100 oder auf dem Boden oder erhöht über dem Boden angebracht sind.

Weiterhin ist eine - in dem in Figur 1 dargestellten Beispiel fest im Boden 100 installierte - Auslesevorrichtung 4 vorgesehen, die die Messdaten - ebenfalls berührungs- und hier insbesondere kabellos - ausliest und an eine Auswertevorrichtung 6 übergibt. Die Auswertevorrichtung 6 wertet unter Anwendung von Algorithmen und/oder Referenzdaten usw. die Messdaten aus und leitet davon Daten ab, die im Zusammenhang mit der Zustandsgröße des Messobjekts 100 stehen. So geben die erzeugten Daten z. B. Auskunft über einen Nitratgehalt im Boden 100. Die erzeugten (Auswerte-)Daten speichert die Auswertevorrichtung 6 hier in einer Speichervorrichtung 5 ab.

Die Speichervorrichtung 5 kann eine zentrale Speichervorrichtung sein, in der die Daten eines Analysesensors - und vorzugsweise aller Analysesensoren - gespeichert werden können. Zusätzlich oder alternativ dazu kann jeder Analysesensor 2 eine eigene Speichereinheit aufweisen. Wenn in diesem Fall der Speicher eines Analysesensors 2 ausfiele, so könnten die einzelnen Analysesensoren 2 miteinander kommunizieren und die Daten des Analysesensors 2 mit defektem Speicher Zwischenspeichern.

Ergänzend ist ein Zusatzsensor 7 vorhanden, der weitere Informationen über das Messobjekt 100 liefert. Dies ist in dem gezeigten Beispiel der pH-Wert. Der Zusatzsensor 7 wird hier ebenfalls von der Auslesevorrichtung 4 ausgelesen, um die Messwerte bezüglich des pH-Werts an die Auswertevorrichtung 6 zu übergeben. Die Messwerte des Zusatzsensors 7 werden somit zusammen mit den Messdaten der Analysesensoren 2 für die Überwachung der Zustandsgröße des Messobjekts 100 - die hier beispielsweise die Belastung des Bodens ist - verwendet.

Die Auslesevorrichtung 4, die Speichervorrichtung 5, die Auswertevorrichtung 6 und der Zusatzsensor 7 können allesamt im Boden 100 eingebracht sein und dort verbleiben.

In dem in Figur 1 abgebildeten Ausführungsbeispiel kommunizieren mindestens zwei - und vorzugsweise alle - Analysesensoren 2 mit der Auslesevorrichtung 4. Das heißt, die Auslesevorrichtung 4 kann mindestens zwei - und vorzugsweise alle - Analysesensoren 2 auslesen. Die ausgelesenen Daten können in der Speichervorrichtung 5 gespeichert werden. Optional können die ausgelesenen Daten von der Auswertevorrichtung 6 ausgewertet werden, und die ausgewerteten Daten können alternativ oder zusätzlich zu den ausgelesenen Daten in der Speichervorrichtung 5 gespeichert werden.

Erfindungsgemäß wird somit ein dauerhaftes eigenständiges Netzwerk innerhalb des Messobjekts 100 geschaffen, wobei die Auslesevorrichtung 4 selbst oder zumindest eine der Auslesevorrichtung 4 zugehörige Ausleseeinheit 4a, einen zentralen Netzwerkknoten zum Auslesen der Analysesensoren 2 bildet. In dem in Figur 1 abgebildeten Ausführungsbeispiel können die Analysesensoren 2 und die Auslesevorrichtung 4 dauerhaft im Boden 100 vergraben sein. Somit wird ein eigenständiges Netzwerk geschaffen, das im Boden 100 vergraben ist und dort dauerhaft verbleiben kann.

Wie eingangs erwähnt, kann die Auslesevorrichtung 4 stationär im Boden 100 angeordnet sein. In diesem Fall könnte beispielsweise ein Kabel an die Oberfläche außerhalb des Bodens geführt sein, um dort stationär die Daten der Auslesevorrichtung 4 und/oder der Speichervorrichtung 5 und/oder der Auswertevorrichtung 6 auszulesen.

Erfindungsgemäß weist die Auslesevorrichtung 4 mindestens zwei Ausleseeinheiten 4a, 4b auf, die drahtlosmiteinander kommunizieren. Dabei ist eine erste Ausleseeinheit 4a, wie in Figur 1 dargestellt, im Boden 100 eingebracht. Eine zweite Ausleseeinheit 4b ist außerhalb des Messobjekts 100 angeordnet und ist als eine mobile Ausleseeinheit ausgestaltet, die wiederum an einem Fahrzeug 12 angeordnet sein kann.

Erfindungsgemäß kommuniziert die mobile zweite Ausleseeinheit 4b mit der im Boden 100 befindlichen Ausleseeinheit 4a. Wie eingangs erwähnt, bildet die erste Ausleseeinheit 4a der Auslesevorrichtung 4 erfindungsgemäß den zentralen Netzwerkknoten, der mindestens zwei - und vorzugsweise alle - Analysesensoren 2 im Boden ausliest. Die zentrale erste Ausleseeinheit 4a kann alle ausgelesenen Daten, ebenfalls zentral, in der damit verbundenen Speichervorrichtung 5 speichern. Somit wird ein eigenständiges Netzwerk innerhalb des Messobjekts 100 geschaffen.

Sobald sich dann die mobile zweite Ausleseeinheit 4b in der Nähe der im Boden 100 vergrabenen ersten Ausleseeinheit 4a befindet, können die beiden Ausleseeinheiten 4a, 4b miteinander kommunizieren und die zuvor mittels der vergrabenen ersten Ausleseeinheit 4a ausgelesenen (bzw. in der Speichervorrichtung 5 gespeicherten) Daten können an die mobile zweite Ausleseeinheit 4b außerhalb bzw. oberhalb des Bodens 100 übertragen werden.

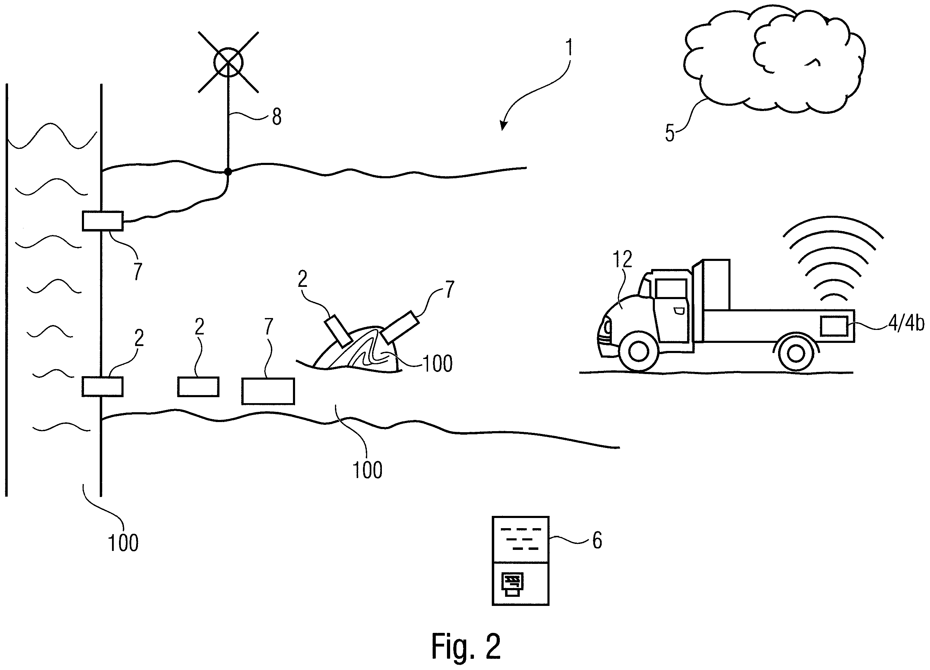

Eine nicht erfindungsgemäße beschriebene Variante sieht vor, dass die Auslesevorrichtung 4 keine separaten im Boden 100 einbringbaren Ausleseeinheiten 4a aufweist, sondern direkt als eine mobile Auslesevorrichtung außerhalb des Bodens 100, beispielsweise an einem Fahrzeug angebracht ist. In diesem Fall würden die im Boden 100 eingebrachten Analysesensoren 2 alle einzeln von der Auslesevorrichtung 4 ausgelesen werden. Eine solches Beispiel wird später mit Bezug auf Figur 2 näher beschrieben.

Die in Figur 1 abgebildete Ausführungsform weist demgegenüber jedoch den Vorteil auf, dass mindestens zwei - und vorzugsweise alle - im Boden vergrabenen Analysesensoren 2 von einer einzelnen ebenfalls im Boden vergrabenen zentralen Ausleseeinheit 4a ausgelesen werden können, und die außerhalb des Bodens 100 angeordnete mobile zweite Ausleseeinheit 4b muss dann lediglich die im Boden vergrabene einzelne zentrale Ausleseeinheit 4a auslesen. Es muss somit also nicht jeder Analysesensor 2 einzeln ausgelesen werden.

Ein weiterer Vorteil besteht darin, dass die Position der im Boden 100 vergrabenen zentralen Ausleseeinheit 4a an der Oberfläche des Bodens 100, zum Beispiel mit einer Fahne oder dergleichen, markiert werden könnte. Bei einem großen Feld 100 könnte der Bauer beispielsweise diese markierte Position direkt anfahren und dort die darunter vergrabene zentrale Ausleseeinheit 4a direkt auslesen. Wenn die Analysesensoren 2 jedoch alle einzeln ausgelesen werden sollen, dann müsste der Bauer das gesamte Feld 100 abfahren, um sichergehen zu können, dass er alle Analysesensoren 2 ausgelesen hat, denn die Analysesensoren 2 können sich überall auf dem Feld befinden. Dies wird vorliegend auch eher der Regelfall sein, denn die Analysesensoren 2 sind als mobile Einheiten konzipiert, die unabhängig voneinander an zufälligen Orten auf dem Feld verteilt werden können. Beim Umpflügen des Felds werden die Analysesensoren 2 sogar noch weiter zufällig verteilt. Vielmehr noch ist eine zufällige Verteilung sogar gewünscht, um Bodenproben an ganz unterschiedlichen Orten des Bodens 100 zu erhalten.

Eine weitere Ausgestaltung des Systems 1 zeigt die Fig. 2. Darin sind insgesamt drei unterschiedliche Messobjekte 100 dargestellt: ein Acker als Beispiel für einen Boden, ein Flusslauf als Beispiel für ein Gewässer und ein Komposthaufen als Beispiel für ein landwirtschaftliches Produkt.

In allen drei Messobjekten 100 ist jeweils ein Analyse-Sensor 2 und jeweils ein Zusatzsensor 7 eingebracht. Der Zusatzsensor 7 des Flusslaufs ist z. B. ein Durchflussmessgerät und der Zusatzsensor 7 des Komposthaufens ist ein Temperaturmessgerät. Der Zusatzsensor 7 des Bodens 100 ermittelt die Feuchtigkeit.

Das Durchflussmessgerät 7 des Gewässers 100 ist hier mit einem Windrad als Beispiel für eine Energiequelle 8 verbunden, die Energy Harvesting betreibt.

Weiterhin ist die Auslesevorrichtung 4 mobil ausgestaltet und wird hier als Beispiel mit einem Traktor 12 durch das Areal bewegt, in dem sich die Messobjekte 100 befinden. Dabei wird insbesondere die Auslesevorrichtung 4 über den Boden als Messobjekt 100 bewegt. Der Auslesevorgang erfolgt dabei beispielweise über eine Anwendung der RFID-Technologie. Die Auslesevorrichtung 4 ist über Funk mit einer Speichervorrichtung 5 verbunden, die hier als Cloud realisiert ist. Wie eingangs erwähnt, weist die Auslesevorrichtung 4 erfindungsgemäß eine erste, fest im Boden 100 installierte, Ausleseeinheit 4a sowie eine mobile zweite Ausleseeinheit 4b auf. Dementsprechend kann es sich bei dem in Figur 2 abgebildeten Beispiel auch um eine an dem Traktor 12 angeordnete zweite Ausleseeinheit 4b handeln, wie sie zuvor unter Bezugnahme auf Figur 1 beschrieben wurde.

Die weitere Auswertung der Messdaten, die in der Speichervorrichtung 5 hinterlegt sind, erfolgt über einen Computer als Auswertevorrichtung 6.

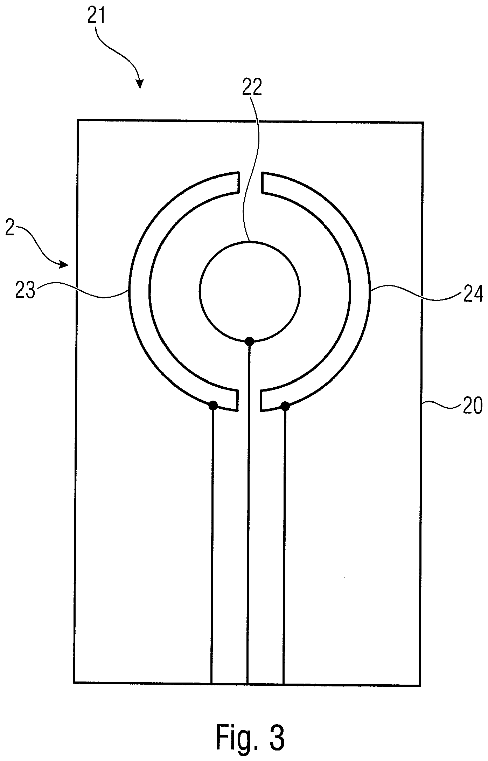

Die Fig. 3 zeigt die ionenselektive Elektrode 21 eines Analysesensors 2, die auf einer flexiblen Folie 20 aufgebracht ist. Über die ionenselektive Elektrode 21 wird die Konzentration eines Ions ermittelt. Hierfür ist - in dem dargestellten Ausführungsbeispiel - eine kreisförmige ionenselektive Membran 22 vorhanden, die umgeben ist von zwei halbkreisförmigen Elektroden in Form einer Referenzelektrode 23 und eine Gegenelektrode 24. Die ionenselektive Membran 22 trennt dabei das - insbesondere feuchte oder zumindest nasse - Messmedium (z. B. das fließende Gewässer oder die Erde des Bodens oder das landwirtschaftliche Produkt selbst) von der Elektrodenanordnung. Die Membran 22 ist über auf- bzw. eingebrachte lonophoren so eingestellt, dass nur die gewünschten Ionen die Membran 22 passieren können. An der Stelle der ionenselektiven Membran 22 befindet sich noch eine Arbeitselektrode, deren elektrische Kontaktierung über eine Leitung dargestellt ist.

Aus der gemessenen elektrischen Spannung oder dem gemessenen elektrischen Potential lässt sich die Konzentration der Ionen ermitteln.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder einer elektronischen Schaltung durchgeführt werden. Bei einigen Ausführungsbeispielen können einige oder mehrere der wichtigsten Verfahrensschritte durch einen solchen Apparat ausgeführt werden.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. System (1) zur Bestimmung und/oder Überwachung mindestens einer Zustandsgröße eines Messobjekts (100),
mit mindestens einem Analysesensor (2), einer Steuervorrichtung (3) und einer Auslesevorrichtung (4),
wobei der Analysesensor (2) ausgestaltet ist, um zum Verbleib in das Messobjekt (100) einbringbar zu sein, und um auf mindestens eine lonenart sensitiv zu sein und ausgehend von einer Konzentration der lonenart im Messobjekt (100) Messdaten zu erzeugen,
wobei die Steuervorrichtung (3) ausgestaltet ist, um den Analysesensor (2) in Bezug auf das Erzeugen der Messdaten zu steuern,
wobei die Auslesevorrichtung (4) ausgestaltet ist, um die Messdaten aus dem Analysesensor (2) auszulesen, und
mit einem dauerhaften eigenständigen Netzwerk mit einem gemeinsamen zentralen Netzwerkknoten,
wobei mehrere Analysesensoren (2) vorhanden sind, die auf die gleiche lonenart oder auf unterschiedliche lonenarten sensitiv sind, wobei die mehreren Analysesensoren (2) ausgestaltet sind, um über den gemeinsamen zentralen Netzwerkknoten auslesbar zu sein,
wobei die Auslesevorrichtung (4) eine erste Ausleseeinheit (4a) und eine zweite Ausleseeinheit (4b) aufweist, wobei die erste Ausleseeinheit (4a) als der gemeinsame zentrale Netzwerkknoten ausgebildet ist, und wobei die zweite Ausleseeinheit (4b) eine außerhalb des Messobjekts (100) angeordnete mobile Ausleseeinheit ist, die ausgestaltet ist, um mit der in dem Messobjekt (100) eingebrachten ersten Ausleseeinheit (4a) drahtlos zu kommunizieren,
wobei die Analysesensoren (2) und die erste Ausleseeinheit (4a) ausgestaltet sind, um dauerhaft im Messobjekt (100) vergraben zu sein,
wobei das System (1) ferner mindestens eine Energiequelle (8) aufweist, die ausgestaltet ist, um mindestens einen Analysesensor (2) mit Energie zu versorgen, wobei die Energiequelle (8) mindestens einen Akku aufweist, und wobei die Energiequelle (8) derartig ausgestaltet ist, dass der Akku induktiv aufladbar ist.

2. System (1) nach Anspruch 1, wobei es sich bei dem Messobjekt (100) um einen Boden, oder um ein Gewässer, oder um ein landwirtschaftliches Produkt handelt.

3. System (1) nach einem der Ansprüche 1 oder 2, wobei die mehreren Analysesensoren (2) ausgestaltet sind, um einzeln auslesbar zu sein.

4. System (1) nach einem der Ansprüche 1 bis 3, wobei mindestens eine Speichervorrichtung (5) vorhanden ist, und wobei die Speichervorrichtung (5) ausgestaltet ist, um die Messdaten und/oder von den Messdaten abgeleitete Daten zu speichern, und wobei die Speichervorrichtung (5) mit der Auslesevorrichtung (4) verbunden und derart ausgestaltet ist, um zum Verbleib in das Messobjekt (100) einbringbar zu sein.

5. System (1) nach einem der Ansprüche 1 bis 4, wobei das System (1) mindestens eine Auswertevorrichtung (6) aufweist, und wobei die Auswertevorrichtung (6) ausgestaltet ist, um die Messdaten im Hinblick auf die Zustandsgröße auszuwerten und/oder von den Messdaten Daten abzuleiten.

6. System (1) nach Anspruch 5, wobei die Auswertevorrichtung (6) mit der Auslesevorrichtung (4) verbunden und derart ausgestaltet ist, um zum Verbleib in das Messobjekt (100) einbringbar zu sein.

7. System (1) nach einem der Ansprüche 1 bis 6, wobei das System (1) mindestens einen Zusatz-Sensor (7) aufweist, wobei der Zusatz-Sensor (7) ein von dem mindestens einen Analysesensor (2) unterschiedlicher ionenunabhängiger Sensor ist, und wobei der Zusatz-Sensor (7) ausgestaltet ist, um zum Verbleib in das Messobjekt (100) einbringbar zu sein und Messdaten mindestens in Abhängigkeit von einer Temperatur, einem Füllstand, einem Durchfluss, einem pH-Wert, einem elektrischen Widerstand, einer elektrischen Leitfähigkeit, einem Anteil eines Gases, einem Anteil von Sauerstoff oder einer Fließgeschwindigkeit zu erzeugen, und wobei der Zusatz-Sensor (7) von der Auslesevorrichtung (4) auslesbar ist.

8. System (1) nach Anspruch 7, wobei das System (1) mindestens eine Auswertevorrichtung (6) aufweist, und wobei die Auswertevorrichtung (6) derartig ausgestaltet ist, um mittels der Auslesevorrichtung (4) ausgelesene Messwerte des ZusatzSensors (7) zusammen mit den Messdaten des mindestens einen Analysesensors (2) für die Überwachung der Zustandsgröße des Messobjekts (100) zu verwenden.

9. System (1) nach einem der vorhergehenden Ansprüche, wobei die Energiequelle (8) ausgestaltet ist, um mittels Energy Harvesting Energie zu erzeugen.

10. System (1) nach einem der Ansprüche 1 bis 9, wobei mindestens ein Analyse-sensor (2) und die Auslesevorrichtung (4) kabellos und/oder durch elektromagnetische Wellen miteinander in Bezug auf Datenübertragung verbunden sind.

11. System (1) nach einem der Ansprüche 1 bis 10, wobei die Auslesevorrichtung (4) als mobile Einheit ausgestaltet ist.

12. System (1) nach einem der Ansprüche 1 bis 11, wobei mindestens ein Analyse-sensor (2) mindestens eine auf einer Folie (20) gedruckte ionenselektive Elektrode (21), eine - vorzugsweise mindestens einen lonophor aufweisende - ionenselektive Membran (22), eine Referenzelektrode (23) und eine Gegenelektrode (24) aufweist.

13. System (1) nach einem der Ansprüche 1 bis 12, wobei mindestens ein Analyse-sensor (2) ausgestaltet ist, um eine Messung mindestens von Nitrat, Nitrit, Chlorid, Fluorid, Sulfat, Ammonium, Sauerstoff, Phosphate, Kalium, Natrium oder Kalzium und/oder deren Abbauprodukte und/oder von mindestens einer Säure und/oder von mindestens einem Abbauprodukt bei einem Gärprozess zu erlauben.

14. System (1) nach einem der Ansprüche 1 bis 13, wobei mindestens ein Analyse-sensor (2) ausgestaltet ist, um eine Messung mindestens von einem Biozid, einem Weichmacher, Algen, Pilzen, Sporen, Bakterien, einem Giftstoff, einem Toxin oder eines metallischen Stoffs erlaubt.

15. System (1) nach einem der Ansprüche 1 bis 14, wobei mindestens ein Analyse-sensor (2) biologisch abbaubar ist.

## Claims

1. System (1) for determining and/or monitoring at least one state variable of a measurement object (100),
comprising at least one analysis sensor (2), a control apparatus (3) and a read-out apparatus (4),
wherein the analysis sensor (2) is configured to be introducible into the measurement object (100) for remaining within the same, and to be sensitive to at least one ion type, and to generate measurement data in the measurement object (100) based on a concentration of the ion type,
wherein the control apparatus (3) is configured to control the analysis sensor (2) with respect to the generation of the measurement data,
wherein the read-out apparatus (4) is configured to read out the measurement data from the analysis sensor (2), and
comprising a permanent individual network with a common central network node, wherein several analysis sensors (2) that are sensitive to the same ion type or to different ion types exist, wherein the several analysis sensors (2) are configured to be read-out via the common central network node,
wherein the read-out apparatus (4) comprises a first read-out unit (4a) and a second read-out unit (4b), wherein the first read-out unit (4a) is configured as the common central network node, and wherein the second read-out unit (4b) is a mobile read-out unit disposed outside the measurement object (100) and configured to communicate wirelessly with the first read-out unit (4a) introduced into the measurement object (100),
wherein the analysis sensors (2) and the first read-out unit (4a) are configured to be permanently buried within the measurement object (100),
wherein the system (1) further comprises at least one energy source (8) that is configured to supply at least one analysis sensor (2) with energy, wherein the energy source (8) comprises at least one accumulator, and wherein the energy source (8) is configured such that the accumulator is chargeable inductively.

2. System (1) according to claim 1, wherein the measurement object (100) is a soil, a body of water, or an agricultural product.

3. System (1) according to claim 1 or 2, wherein the several analysis sensors (2) are configured to be read out individually.

4. System (1) according to claim 1 to 3, wherein at least one memory apparatus (5) exists, and wherein the memory apparatus (5) is configured to store the measurement data and/or data derived from the measurement data, and wherein the memory apparatus (5) is connected to the read-out apparatus (4) and configured in order to be introducible into the measurement object (100) for remaining within the same.

5. System (1) according to claim 1 to 4, wherein the system (1) comprises at least one evaluation apparatus (6), and wherein the evaluation apparatus (6) is configured to evaluate the measurement data with respect to the state variable and/or to derive data from the measurement data.

6. System (1) according to claim 5, wherein the evaluation apparatus (6) is connected to the read-out apparatus (4) and configured in order to be introducible into the measurement object (100) for remaining within the same.

7. System (1) according to any of claims 1 to 6, wherein the system (1) comprises at least one additional sensor (7), wherein the additional sensor (7) is an ion-independent sensor differing from the at least one analysis sensor (2), and wherein the additional sensor (7) is configured to be introducible into the measurement object (100) for remaining within the same, and to generate measurement data at least in dependence on a temperature, fill level, flow rate, pH value, electric resistance, electric conductivity, proportion of a gas, proportion of oxygen or flow velocity, and wherein the additional sensor (7) can be read out by the read-out apparatus (4).

8. System (1) according to claim 7, wherein the system (1) comprises at least one evaluation apparatus (6), and wherein the evaluation apparatus (6) is configured to use measurement values of the additional sensor (7) read out by the read-out apparatus (4) together with the measurement data of the at least one analysis sensor (2) for monitoring the state variable of the measurement object (100).

9. System (1) according to any of the preceding claims, wherein the energy source (8) is configured to generate energy by means of energy harvesting.

10. System (1) according to any of claims 1 to 9, wherein at least one analysis sensor (2) and the read-out apparatus (4) are connected wirelessly and/or by electromagnetic waves with regard to data transmission.

11. System (1) according to any of claims 1 to 10, wherein the read-out apparatus (4) is configured as a mobile unit.

12. System (1) according to any of claims 1 to 11, wherein at least one analysis sensor (2) comprises at least one ion-selective electrode (21) printed on a foil (20), an ion-selective membrane (22) preferably comprising at least one ionophore, a reference electrode (23) and a counter electrode (24).

13. System (1) according to any of claims 1 to 12, wherein at least one analysis sensor (2) is configured to allow a measurement of at least nitrate, nitrite, chloride, fluoride, sulfate, ammonium, oxygen, phosphate, potassium, sodium or calcium and/or the degradation products of the same and/or at least one acid and/or at least one degradation product during a fermentation process.

14. System (1) according to any of claims 1 to 13, wherein at least one analysis sensor (2) is configured to allow a measurement of at least one biocide, plasticizer, algae, fungi, spores, bacteria, a toxic agent, a toxin or a metallic substance.

15. System (1) according to any of claims 1 to 14, wherein at least one analysis sensor (2) is biodegradable.

## Revendications

1. Système (1) de détermination et/ou de surveillance d'au moins une grandeur d'état d'un objet de mesure (100),
avec au moins un capteur d'analyse (2), un dispositif de commande (3) et un dispositif de lecture (4),
dans lequel le capteur d'analyse (2) est conçu de manière à pouvoir être introduit dans l'objet de mesure (100) pour y rester et à être sensible à au moins un type d'ions et à générer des données de mesure en partant d'une concentration de type d'ions dans l'objet de mesure (100),
dans lequel le dispositif de commande (3) est conçu pour commander le capteur d'analyse (2) en ce qui concerne la génération des données de mesure,
dans lequel le dispositif de lecture (4) est conçu pour lire les données de mesure du capteur d'analyse (2), et
avec un réseau autonome permanent avec un nœud de réseau central commun,
dans lequel sont présents plusieurs capteurs d'analyse (2) qui sont sensibles au même type d'ions ou à des types d'ions différents, dans lequel les plusieurs capteurs d'analyse (2) sont conçus pour pouvoir être lus via le nœud de réseau central commun,
dans lequel le dispositif de lecture (4) présente une première unité de lecture (4a) et une deuxième unité de lecture (4b), dans lequel la première unité de lecture (4a) est conçue comme le nœud de réseau central commun, et dans lequel la deuxième unité de lecture (4b) est une unité de lecture mobile disposée à l'extérieur de l'objet de mesure (100) qui est conçue pour communiquer sans fil avec la première unité de lecture (4a) disposée dans l'objet de mesure (100),
dans lequel les capteurs d'analyse (2) et la première unité de lecture (4a) sont conçus pour être noyés de manière permanente dans l'objet de mesure (100),
dans lequel le système (1) présente par ailleurs au moins une source d'énergie (8) qui est conçue pour alimenter en énergie au moins un capteur d'analyse (2), dans lequel la source d'énergie (8) présente au moins une batterie rechargeable, et dans lequel la source d'énergie (8) est conçue de sorte que la batterie rechargeable puisse être chargée par induction.

2. Système (1) selon la revendication 1, dans lequel il s'agit, pour l'objet de mesure (100), d'un sol, ou d'une étendue d'eau, ou d'un produit agricole.

3. Système (1) selon l'une des revendications 1 ou 2, dans lequel les plusieurs capteurs d'analyse (2) sont conçus pour pouvoir être lus individuellement.

4. Système (1) selon l'une des revendications 1 à 3, dans lequel est présent au moins un dispositif de mémoire (5), et dans lequel le dispositif de mémoire (5) est conçu pour mémoriser les données de mesure et/ou les données dérivées des données de mesure, et dans lequel le dispositif de mémoire (5) est connecté au dispositif de lecture (4) et est conçu de sorte qu'il puisse être introduit dans l'objet de mesure (100) pour y rester.

5. Système (1) selon l'une des revendications 1 à 4, dans lequel le système (1) présente au moins un dispositif d'évaluation (6), et dans lequel le dispositif d'évaluation (6) est conçu pour évaluer les données de mesure en ce qui concerne la grandeur d'état et/ou dériver les données des données de mesure.

6. Système (1) selon la revendication 5, dans lequel le dispositif d'évaluation (6) est connecté au dispositif de lecture (4) et est conçu de sorte qu'il puisse être introduit dans l'objet de mesure (100) pour y rester.

7. Système (1) selon l'une des revendications 1 à 6, dans lequel le système (1) présente au moins un capteur additionnel (7), dans lequel le capteur additionnel (7) est un capteur indépendant des ions différent de l'au moins un capteur d'analyse (2), et dans lequel le capteur additionnel (7) est conçu pour pouvoir être introduit dans l'objet de mesure (100) pour y rester et pour générer des données de mesure au moins en fonction d'une température, d'un niveau de remplissage, d'un débit, d'une valeur de pH, d'une résistance électrique, d'une conductivité électrique, d'une part d'un gaz, d'une part d'oxygène ou d'une vitesse de débit, et dans lequel le capteur additionnel (7) peut être lu par le dispositif de lecture (4).

8. Système (1) selon la revendication 7, dans lequel le système (1) présente au moins un dispositif d'évaluation (6), et dans lequel le dispositif d'évaluation (6) est conçu de manière à utiliser les valeurs de mesure du capteur additionnel (7) lues au moyen du dispositif de lecture (4) ensemble avec les données de mesure de l'au moins un capteur d'analyse (2) pour la surveillance de la grandeur d'état de l'objet de mesure (100).

9. Système (1) selon l'une des revendications précédentes, dans lequel la source d'énergie (8) est conçue pour générer de l'énergie au moyen d'un Energy Harvesting (= Captage d'Energie).

10. Système (1) selon l'une des revendications 1 à 9, dans lequel au moins un capteur d'analyse (2) et le dispositif de lecture (4) sont connectés l'un à l'autre sans fil et/ou par des ondes électromagnétiques en ce qui concerne la transmission de données.

11. Système (1) selon l'une des revendications 1 à 10, dans lequel le dispositif de lecture (4) est conçu comme une unité mobile.

12. Système (1) selon l'une des revendications 1 à 11, dans lequel au moins un capteur d'analyse (2) présente, imprimée sur un film (20), au moins une électrode sélective d'ions (21), une membrane sélective d'ions (22) - présentant de préférence au moins un ionophore -, une électrode de référence (23) et une contre-électrode (24).

13. Système (1) selon l'une des revendications 1 à 12, dans lequel au moins un capteur d'analyse (2) est conçu pour permettre une mesure au moins de nitrate, de nitrite, de chlorure, de fluorure, de sulfate, d'ammonium, d'oxygène, de phosphates, de potassium, de sodium ou de calcium et/ou de leurs produits de décomposition et/ou d'au moins un acide et/ou d'au moins un produit de décomposition lors d'un processus de fermentation.

14. Système (1) selon l'une des revendications 1 à 13, dans lequel au moins un capteur d'analyse (2) est conçu pour permettre une mesure d'au moins un biocide, d'un agent adoucissant, d'algues, de champignons, de spores, de bactéries, d'un poison, d'une toxine ou d'un matériau métallique.

15. Système (1) selon l'une des revendications 1 à 14, dans lequel au moins un capteur d'analyse (2) est biodégradable.
